# EUROPEAN PATENT SPECIFICATION

(11) **EP 1 642 565 B1**
(45) Date of publication and mention of the grant of the patent: **21.07.2010**
(21) Application number: 04022405.7
(22) Date of filing: 21.09.2004
(51) Int. Cl.: A61K 8/41, A61Q 5/04, A61K 8/04, A61K 8/46, A61K 8/44

(54) **Product for permanent shaping of keratin fibres packaged in a two chamber aerosol can**
Haardauerwellmittel in einer Sprühdose mit zwei getrennten Kammern
Produit pour une permanente des cheveux dans un aérosol à deux chambres

(43) Date of publication of application: 05.04.2006
(73) Proprietor: KPSS-Kao Professional Salon Services GmbH, 64297 Darmstadt (DE)
(72) Inventor: Lamboy, Peter, 64331 Weiterstadt (DE); Vogelsang, Herbert, 64297 Darmstadt-Eberstadt (DE)

(56) References cited:
- EP-A- 0 269 068
- WO-A-03/091128
- DE-A- 19 717 080
- US-A- 4 842 849
- US-A- 5 775 549
- US-A1- 2003 024 542

## Description

This invention relates to a process for obtaining a permanent shaping product for keratin fibres consisting of a two chamber pressurised can and a ready to use aqueous composition comprising at least one reducing agent.

It is generally known that permanent shaping is carried out in two steps, the reductive splitting of the cystein disulfide bonds in the hair by a reducing agent and the subsequent neutralisation by application of an oxidizing agent, whereby the cystein disulfide bonds are restored.

The reducing agent used still most frequently is thioglycolic acid also in the form of the salts thereof, in particular its ammonium salt, although numerous other thio compounds have been proposed for this purpose which, however, mostly have not been successfully applied because of toxicological reasons. The reducing agents especially of interest are thiogylcolic acid, cystein, thiolactic acid and cystamine. Another one is glyceryl monothiogylcolate (GMTG) showing very good permanent shaping effect however has some toxicological problems. The reducing agents are in general used at a concentration of 1 to 20% by weight, preferably 1 to 15% by weight. It should be noted that regulations on the concentration of reducing agents must be followed in those countries where authorities do so.

Compositions for permanent shaping hair are usually packed as single dose units because of their sensibility to oxidation when contacting air. Multi-portion products are as well available although the great stability concerns. Once products are opened and emptied partly, remaining product is losing its effect as a reducing agent and this certainly affects the results of the permanent shaping process. Therefore, there is a great need for multi-portion products for permanent shaping hair. Products as single dose units bring certainly high packaging costs and waste, which is, in addition, not always environmental friendly. Additionally, usually because of stability reasons, they have to be packed into a multi phase products, which are mixed prior to application. This requires as well expensive special packaging technology, which brings certainly limitations in practice.

It is, therefore, highly desirable to achieve ready to use products, without any mixing step prior to use, for permanent shaping hair, especially from the view point of a multi-portion product, with low cost without loosing the performance in terms of stability and permanent shaping ability during whole life time of the product. This invention starts form the problems as explained and aims primarily for finding an alternative, which is more cost effective, environmental friendly and as well be packed without using flammable gas. The alternative packaging makes withdrawal of content of package in smaller portions than the whole content of the product without affecting the stability of the remaining product.

US 5775549 and DE 19717080 A1 disclose spray package comprising a piston to be used in the fields of foodstuffs, chemistry, cosmetics, dermatology, insecticides or cleaning agents. Nothing is said about a reducing composition for permanent shaping hair in both documents.

US 4842849 discloses composition intended fro the treatment of keratin fibres based on a cationic polymer and an anionic polymer containing vinylsulphonic groups. According to the document, compositions can be packaged as an aerosol in which they can be applied either in the form of an aerosol spray or as an aerosol foam. Reducing compositions are disclosed for permanent shaping hair without a disclosure of an aerosol confectioning. Document is silent on an aerosol can with a piston.

It has now been found out that a multiportion permanent shaping ready to use product can be achieved by using a pressurised can containing two chambers separated with a movable unsupported piston. This form of preparation show advantages over the conventional aerosol products readily available on the market as permanent shaping composition is not mixed with any propellant gas it does not come out from the packaging in the form of foam or any form of enlarged volume. It is readily available as a composition without any gas so that foam breakage or any other points are not need to be concerned when developing compositions.

With the term permanent shaping throughout the description and as well with the claims, the reference is made to perming and straightening processes.

The inventors have surprisingly found out that a process for obtaining a permanent hair shaping product, using a two-chamber can (1) consisting of an outer wall (2) having a closure (3) with a valve (5) and a bottom (4) with a stopper (6) which contains a vertically movable unsupported piston (7) with a flexible silicone coverage (12) and the support (11), the movable piston (7) divides the whole volume of the can into two completely separate volumes (8 and 9) one (8) being filled with an aqueous permanent shaping composition and the other (9), being the lower part, is filled with an oxygen free non-flammable gas to build up pressure in the can is first placed in a filling place
b- by means of extendable filling nozzle entering the can through the closure area, the movable piston (7) is pushed to the very bottom of the can which is followed by purging the can content with an oxygen free gas, preferably same as the pressurising gas and is selected from nitrogen and carbon dioxide, through a nozzle which may be same or different to the filling nozzle,
c- through the opening (6) at the bottom (4) the lower part of the can is purged with oxygen free gas, preferably same as the pressurising gas and is selected from nitrogen and carbon dioxide,
d- pushing the piston (7) down again through the opening with an extendable nozzle and filling the aqueous composition comprising at least one reducing agent already produced into the upper part of the can (8),
e- purging the upper space of the can left after filling the composition again with an oxygen free non-flammable gas,
f- placing the closure (3) and valve (5) on top of the can, and
g- pressurised gas is filled into to lower part (9) and stopper (plug) (6) is placed.
The can makes storage of a permanent shaping composition for hair for a long period of time. When the content of such a can is only partially emptied, the remaining is stored without showing any signs of instability for a long time. Such kind of packaging materials are commercially available.

It has as well been found out that the steps b and c can be exchanged meaning that the step c can be carried out before the step b and then continued with step b and step d etc.

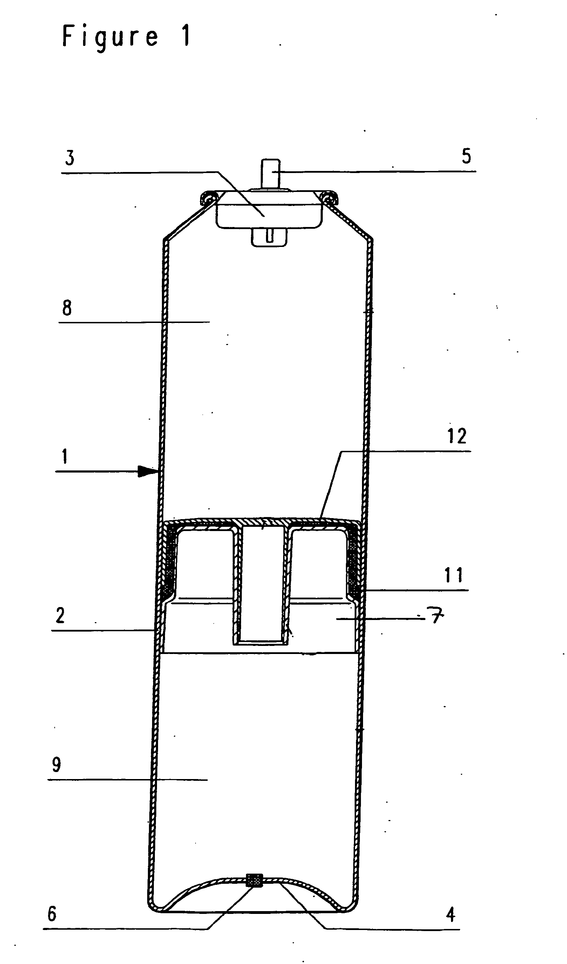

Figure 1 represents schematically pressurised can the numbers correspond to the same as above.

Composition filled into the can to obtain product is an aqueous permanent shaping composition for hair comprising at least one reducing agent. The reducing agents used are thiogylcolic acid, cystein, thiolactic acid, cystamine, glycerin monothiogylcolate. Thiogylcolic acid, cystein and thiolactic acid or mixtures are the most preferred ones at a concentration of 1 to 20% by weight and preferably 1 to 15% by weight. The regulations from the authorities should be taken into consideration concerning the concentration of individual reducing agent in certain countries.

The pH of the permanent shaping composition is in the range of 5 to 10 preferably 6 to 10 and more preferably 6.5 to 9.5 and most preferably 6.8 to 9.5. The pH of the composition is adjusted preferably with ammonium hydroxide or monoethanolamine. Ammonium carabamate and/or sodium bicarbonate are as well used for pH adjustment, in certain case even preferred because of their buffering capacities. Both agents are not used in acidic pH ranges. Organic and or inorganic acids may as well be used for adjusting the pH in addition to the alkalising agents mentioned above.

According to the invention, composition for permanent shaping hair is an aqueous solution or dispersion either clear or opaque, which can further be an emulsion or a gel. The solution and dispersion forms with clear or opaque appearance are the most preferred.

The viscosity of the compositions according to the invention is preferably below 5,000, in particular below 3,000, especially below 1,000 mPa.s, measured at 20°C with a Brookfield rotation viscosimeter with an appropriate spindle.

Permanent shaping composition of the present invention can comprise surfactants of anionic, non-ionic, amphoteric or zwitterionic and/or cationic surfactants or their mixtures at a concentration of 0.1 to 10%, preferably 0.1 to 7.5% and more preferably 0.1 to 5% by weight, calculated to the total composition. The surfactants, especially the nonionic ones, are especially used for solubilizing aid for insoluble lipophilic compounds such as fragrance and oily conditioning compounds.

Suitable anionic surfactants are of the sulfate, sulfonate, carboxylate and alkyl phosphate type, especially, of course, those customarily used in shampoo compositions, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates constituting mild, skin-compatible detergents.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₁-(C₂H₄O)ₙ-O-CH₂COOX,

wherein R₁ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 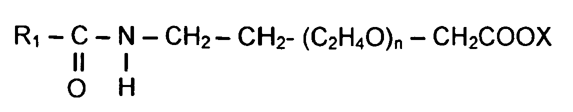 wherein R₁ and X have the above meanings, and n is in particular a number from 1 to 10, preferably 2.5 to 5.

Such products have been known for some time and are on the market, for example, under the trade name "AKYPO®" and "AKYPO-SOFT®".

Also useful are C₈-C₂₀-acyl isethionates, alone or in admixture with other anionic surfactants, as well as sulfofatty acids and the esters thereof.

An overview of the anionic surfactants suiutable for compositions of the present invention can furthermore be found in the monography of K. Schrader, "Grundlagen und Rezepturen der Kosmetika", 2nd Ed.(1989, Hüthig Buchverlag), pp. 595-600 and pp. 683 to 691.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

Further surfactants in the permanent shaping compositions according to the invention are nonionic surfactants, which are especially preferred as solubilizing aid for lipophilic water insoluble compounds. Especially suited are alkyl polyglucosides of the general formula

R₂-O-(R₃O)ₙ-Zₓ,

wherein R₂ is an alkyl group with 8 to 18 carbon atoms, R₃ is an ethylene or propylene group, Z is a saccharide group with 5 to 6 carbon atoms, n is a number from 0 to 10 and x is a number between 1 and 5.

Further nonionic surfactant components are, for example, long-chain fatty acid mono- and dialkanolamides, such as coco fatty acid monoethanolamide and myristic fatty acid monoethanolamide.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Such amineoxides are state of the art, for example C₁₂-C₁₈- alkyl dimethyl amineoxides such as lauryl dimethyl amineoxide, C₁₂-C₁₈- alkyl amidopropyl or -ethyl amineoxides, C₁₂-C₁₈ -alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx®", "Aromox®" or "Genaminox®".

Further nonionic surfactants useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

As solubilizing aid especially preferred are the ethoxylated hydrogenated castor oil known with the trade name Cremophor from the company BASF.

As further surfactant component, the compositions according to the invention can also contain amphoteric or zwitterionic surfactants. Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, it is possible to use betaines of the structure 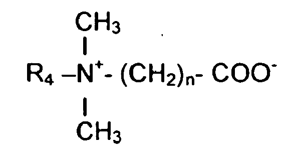 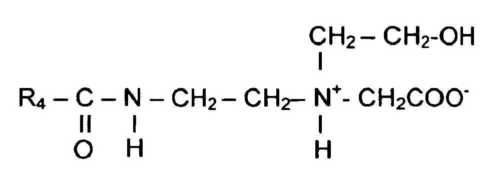 wherein R₄ is a C₈-C₁₈-alkyl group and n is 1 to 3;
sulfobetaines of the structure 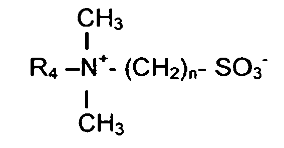 wherein R₄ and n are same as above;
and amidoalkyl betaines of the structure 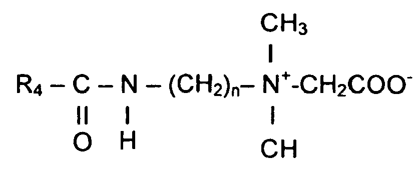 wherein R₄ and n are same as above.

Permanent shaping compositions of the present invention can comprise additionally one or more cationic surfactants, especially as conditioner, presented with the general formula 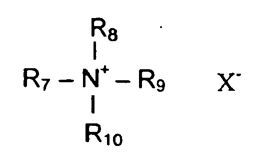 where R₇ is a saturated or unsaturated, branched or non-branched alkyl chain with 8-22 C atoms or

R₁₁ CO NH (CH₂)n

where R₁₁ is saturated or unsaturated, branched or non-branched alkyl chain with 7-21 C atoms and n has value of 1 - 4,
or

R₁₂ CO O (CH₂)n

where R₁₂ is saturated or unsaturated, branched or non-branched alkyl chain with 7-21C atoms and n has value of 1 - 4, and
R₈ is H or unsaturated or saturated, branched or non-branched alkyl chain with 1 - 4 C atoms or

R₁₁ CO NH (CH₂)ₙ

or

R₁₂ CO O (CH₂)n

where R₁₁, R₁₂ and n are same as above.

R₉ and R₁₀ are H or lower alkyl chain with 1 to 4 carbon atoms, and X is anion such as chloride, bromide, methosulfate.

Typical examples of those ingredients are cetyl trimethly ammonium chloride, stear trimonium chloride, behentrimoinium chloride, stearamidopropyl trimonuim chloride, dioleoylethyl dimethyl ammonium methosulfate, dioleoylethyl hydroxyethylmonium methosulfate.

The composition of the present invention comprises further hair-conditioning agents. Conditioning agents can be selected from oily substances, non-ionic substances, cationic polymers or their mixtures. Oily substances are selected from such as silicone oils, either volatile or non-volatile, natural and synthetic oils. Among silicone oils those can be added to the pre-treatment composition include dimethicone, dimethiconol, polydimethylsiloxane, DC fluid ranges from Dow Corning, natural oils such as olive oil, almond oil, avocado oil, weizenkeim oil, ricinus oil and the synthetic oils, such as mineral oil.

Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₁₃ CO (O CH₂ CH₂)n OH

R₁₃ CO (O CH₂ CH₂)ₙ O OC R₁₄

where R₁₃ and R₁₄ are independent from each other saturated, unsaturated or branched or non-branched alkyl chain with 7 to 21 C atoms and n is typically 2 - 100.

Permanent shaping compositions of the present invention can contain additionally cationic polymers as conditioning agents. Suitable polymers are those of cationic polymers best known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-72, Quaternium-78, Quaternium-80, Quaternium-81, Quaternium-81, Quaternium-82, Quaternium-83 and Quaternium-84. It has been found out that Quaternium-80 is the best suitable one among the Quaterniums.

It has further been found out that especially those of cationic cellulose type polymers known as Polymer JR type from Amerchol such as Polyquaternium 10 or cationic guar gum known with trade name Jaguar from Rhône-Poulenc and chemically for example Guar hydroxypropyl trimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

Composition can comprise an organopolysiloxane wherein at least one silicium atom is linked to an alkylene group having a hetero-atom, in particular a nitrogen atom, with a poly-(N-acyl alkyleneimine) units of the formula 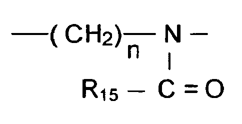 wherein n is a number from 1 to 5 and R₁₅ is hydrogen, a C₁-C₁₂-alkyl or cycloalkyl, aralkyl or aryl group.

Preferred organopolysiloxane polymers are those of the type disclosed in EP-A 640 643, in particular optionally quaternized aminoalkyl, in particular aminopropyl dimethyl polysiloxane/polyethyl oxazoline copolymers of the formula 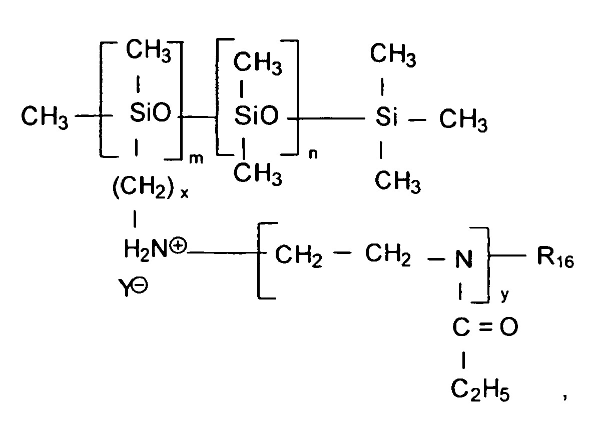 wherein m and n each are numbers from 20 to 10,000, in particular 50 to 7,000, especially 100 to 5,000, x is a number between 1 and 5, preferably 3, and y is a number from 5 to 30, R₁₆ is a C₁-C₁₂-alkyl or aryl group, in particular a methyl, ethyl or benzyl group, and Y- is an anion.

Especially suited are the organopolysiloxanes disclosed under the terms A-1, A-2 and A-3 on pages 12 to 13 of EP-A 640 643. The proportion of graft copolymers in the hair colouring compositions according to the invention ranges from 0.05 % to 5 %, preferably 0.1 % to 2.5 %, in particular 0.5 % to 1.5 % by weight, calculated to the total composition.

Composition according to the present invention can contain organic solvents as penetration enhancers and also as a solubilzers. Examples of such organic solvents are benzyloxy ethanol, benzyl alcohol, phenoxy ethanol, phenoxy isopropanol, methyl phenoxy ethanol, benzyl glycerol, N-benzyl formide, N-methyl pyrrolidone, N-ethyl pyrrolidone, cinnamyl alcohol, phenethyl alcohol, p-methyl benzyl alcohol, butyl cellosolve, methyl carbitol, ethyl carbitol, propyl carbitol, butyl carbitol, diethyleneglycol, diethyl ether and dipropyleneglycol diethyl ether. Typically the concentration of those solvents can be in the range from 0.5% to 25%, preferably 0.5 - 20% by weight and more preferably 0.5 - 15% by weight, calculated to the total composition.

Urea is as well preferred as penetration enhancer.

Permanent shaping compositions according to the invention can contain thickening agents, especially in the case a gel type of preparation is preferred. These are, for example, the various cellulose derivatives such as hydroxyalkyl celluloses, e.g. hydroxyethyl cellulose, hydroxypropyl cellulose, hydroxypropyl methyl cellulose, natural polysaccharides such as xanthan gum; guar gum and the alkoxylation products thereof in amounts from 0.1 - 5 %, preferably 0.1 - 3% and most preferably 0.1 - 2% by weight calculated to the total composition and depending on the desired consistency thereof.

Another compound may be used in the composition is of ceramide type of compounds according to general formula 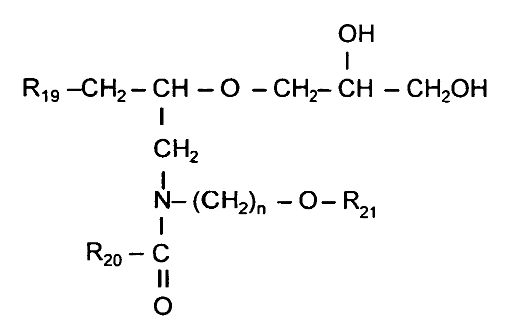 where R₁₉ and R₂₀ are independent from each other alkyl- or alkenyl group mit 10 to 22 carbon atoms, R₂₁ is methyl, ethyl, n-propyl or isopropyl group and n is a number between 1 to 6, preferably 2 or 3. The concentration of ceramide type of compound in colouring compositions of the present invention can be in the range of 0.01 to 2 and especially 0.01 to 1% by weight calculated to the total composition.

The product according to the present invention comprises at least one gas. Gas is an oxygen free and non-flammable and is selected from nitrogen, carbon dioxide and dinitrogen oxide (N₂O). The pressure within the can should not exceed 15 bar, preferably 10 bar and should be at least 1 bar preferably 3 and more preferably 5 bar bar. The amount of oxygen free, non-flammable gas is determined according to required pressure inside can.

The cans can be emptied with very low level of remains in the can. The emptying is achieved at most not les than 98% of its content by weight.

The following examples are used to illustrate the invention, but not limit it

### Examples

### Example 1

| | % by weight |
|---|---|
| Ammonium thiogylcolate | 9.5 |
| Ammonium hydroxide (25%) | 6.6 |
| Ammonium carbonate | 1.0 |
| Cremophore RH 60* | 1.5 |
| C12-C16 alkyl polyglucoside | 1.5 |
| Fragrance | q.s |
| Water | to 100 |

| | |
|---|---|
| *Ethoxylated hydrogenated castor oil | |

The composition of example 1 was filled into the can as described above. Nitrogen gas is used to achieve a pressure of 8 bar inside the can. The product was stable over the period of 12 weeks (period tested) at 40°C and at room temperature. No instability is observed in terms of perming performance on hair, which was shown in a comparative permanent shaping test on hair switches, where the permanent shaping was carried out with a product as above aged and a freshly prepared composition. In the perming process, as an oxidation agent 2.3% by weight hydrogen peroxide comprising oxidizing agent is used.

In another run, the filling into the cans was carried out as described above except that the filling the step 3 as described above was not carried out, namely the lower part (9) was not purged with an oxygen free, non-flammable gas and the stability of the product was tested. After storing 2 weeks at 40°C and at room temperature, the above-mentioned test was carried out again. This result showed clearly that there was significant decrease in reducing performance, this was due to the oxidative damage of reducing agent.

These results show clearly that the above-described procedure is very important in order to guarantee the optimum stability of the product for the whole shelf life.

Similar results are obtained with the following examples as well.

### Example 2

| | % by weight |
|---|---|
| Ammonium thiogylcolate | 5.0 |
| Cystein | 2.5 |
| Ammonium hydroxide (25%) | 3.2 |
| Cremophore RH 60* | 1.5 |
| Oleyl polyglucoside | 0.5 |
| Fragrance | q.s |
| Water | to 100 |

| | |
|---|---|
| *Ethoxylated hydrogenated castor oil | |

The preparation and filling and perming tests were carried out as above. No instability was observed.

### Example 3

| | % by weight |
|---|---|
| Thiolactic acid | 12.5 |
| Ammonium hydroxide (25%) | 9.0 |
| Ammonium bicarbonate (25%) | 5.0 |
| Urea | 4.0 |
| Polyquatenium-6 | 0.5 |
| Cocbetaine | 0.8 |
| Fragrance | q.s |
| Water | to 100 |

The preparation and filling and perming tests were carried out as above. No instability was observed.

## Claims

1. Process for obtaining permanent shaping product consisting of a pressurised can and an aqueous composition comprising at least one reducing agent **characterised in that**
a- the can (1) consisting of an outer wall (2) having a closure (3) with a valve (5) and a bottom (4) with a stopper (6) which contains a vertically movable unsupported piston (7) with a flexible silicone coverage (12) and the support (11), the movable piston (7) divides the whole volume of the can into two completely separate volumes (8 and 9) one (8) being filled with an aqueous permanent shaping composition and the other (9), being the lower part, is filled with an oxygen free non-flammable gas to build up pressure in the can is first placed in a filling place,
b- by means of extendable filling nozzle entering the can through the closure area, the movable piston (7) is pushed to the very bottom of , the can which is followed by purging the can content with an oxygen free gas, preferably same as the pressurising gas and is selected from nitrogen and carbondioxide, through a nozzle which may be same or different to the filling nozzle,
c- through the opening (6) at the bottom (4) the lower part of the can is purged with oxygen free gas, preferably same as the pressurising gas and is selected from nitrogen and carbondioxide,
d- pushing the piston (7) down again through the opening with an extendable nozzle and filling the aqueous composition comprising at least one reducing agent already produced into the upper part of the can (8),
e- purging the upper space of the can left after filling the composition again with an oxygen free non-flammable gas,
f- placing the closure (3) and valve (5) on top of the can, and
g- pressurised gas is filled into to lower part (9) and stopper (plug) (6) is placed.

2. Process according to claim 1 **characterised in that** the steps b and c are carried out in the opposite order.

3. Process according to claims 1 and 2 **characterised in that** oxygen free non-flammable gas is selected from nitrogen, carbon dioxide and dinitrogen oxide (N₂O).

4. Process according to claims 1, 2 and 3 **characterised in that** reducing agent in aqueous composition is selected form thiogylcolic acid, cystein, thiolactic acid and cystamine.

5. Process according to claims 1 to 4 **characterised in that** aqueous composition comprising at least one reducing agent has a viscosity lower than 5,000 mPa.s measured at 20°C with a Brookfiled viscosimeter.

6. Process according to any of the preceding claims **characterised in that** aqueous composition comprising at least one reducing agent has a pH in the range of 5 to 10.

7. Process according to any of the preceding claims **characterised in that** aqueous composition comprising at least one reducing agent comprises at least one surfactant selected from anionic, non-ionic, amphoteric and cationic ones.

8. Process according to any of the preceding claims **characterised in that** aqueous composition comprising at least one reducing agent comprises at least one organic solvent as a penetration enhancer and/or urea.

9. Process according to any of the preceding claims **characterised in that** aqueous composition comprising at least one reducing agent comprises at least one hair conditioning agent.

## Patentansprüche

1. Verfahren zur Erstellung eines Produkts zur permanenten Umverformung, bestehend aus einer unter Druck stehenden Dose und einer wässrigen Zusammensetzung, die mindestens ein Reduktionsmittel enthält, **dadurch gekennzeichnet, dass**
a- die Dose (1), bestehend aus einer äußeren Wand (2), die einen Verschluss (3) mit einem Ventil (5) und einen Boden (4) mit einem Stopfen (6) hat, in der sich ein vertikal beweglicher, nicht unterstützter Kolben (7) mit einer flexiblen Siliconbeschichtung (12) und der Unterstützung (11) befindet, der bewegliche Kolben (7) des weiteren das gesamte Volumen der Dose in zwei komplett separate Volumen (8 und 9) trennt, Teil (8) gefüllt mit der wässrigen permanenten Umformungs- Zusammensetzung und der andere Teil (9), welcher der untere Teil ist, mit einem Sauerstoff freien, nicht brennbaren Gas gefüllt ist, um den Druck in der Dose aufzubauen, die Dose zuerst in einer Füllstation platziert wird und
b- mittels einer einschiebbaren Fülltülle, die durch den Verschlussbereich in die Dose eintritt, der bewegliche Kolben (7) ganz nach unten auf den Boden der Dose gedrückt wird, gefolgt durch Purging (ausblasen) des Dosenvolumens mit einem Sauerstoff freien Gas, vorzugsweise dem gleichen wie das Druck gebende Gas, ausgewählt aus Stickstoff und Kohlendioxid durch eine Tülle, welche die gleiche oder auch eine andere wie die Fülltülle sein kann,
c- durch die Öffnung (6) am Boden (4) der untere Teil der Dose mit einem Sauerstoff freien Gas, vorzugsweise dem gleichen wie das Druck gebende Gas, ausgewählt aus Stickstoff und Kohlendioxid, gepurged (ausgeblasen) wird,
d- der Kolben (7) nochmals mit der einschiebbaren Tülle nach unten gedrückt wird und mit der bereits produzierten wässrigen Zusammensetzung, die mindestens ein Reduktionsmittel enthält, die Dose im oberen Teil (8) befüllt wird,
e- der oberste Raum der Dose, der nach dem Befüllen mit der Zusammensetzung verblieben ist, nochmals mit einem Sauerstoff freien Gas gepurged (ausgeblasen) wird,
f- der Verschluss (3) und das Ventil (5) auf den oberen Teil der Dose aufgebracht wird, und
g- das Druck gebende Gas in den unteren Teil (9) gefüllt und der Stopfen (6) eingebracht wird.

2. Verfahren nach Anspruch 1, **dadurch gekennzeichnet, dass** die Schritte b und c in umgekehrter Reihenfolge durchgeführt werden.

3. Verfahren nach den Ansprüchen 1 und 2, **dadurch gekennzeichnet, dass** das Sauerstoff freie, nicht brennbare Gas aus Stickstoff, Kohlendioxid und Distickstoffoxid (N₂O) ausgewählt ist.

4. Verfahren nach den Ansprüchen1, 2 und 3, **dadurch gekennzeichnet, dass** das Reduktionsmittel in der wässrigen Zusammensetzung aus Thioglycolsäure, Cystein, Thiomilchsäure und Cystamine ausgewählt ist.

5. Verfahren nach den Ansprüchen 1 bis 4, **dadurch gekennzeichnet, dass** die wässrige Zusammensetzung, die mindestens ein Reduktionsmittel enthält, eine Viskosität unter 5.000 mPa.s hat, gemessen mit einem Brookfield- Viskosimeter bei 20°C.

6. Verfahren nach einem der vorhergehenden Ansprüche, **dadurch gekennzeichnet, dass** die wässrige Zusammensetzung, die mindestens ein Reduktionsmittel enthält, einen pH- Wert im Bereich von 5 bis 10 hat.

7. Verfahren nach einem der vorhergehenden Ansprüche, **dadurch gekennzeichnet, dass** die wässrige Zusammensetzung, die mindestens ein Reduktionsmittel enthält, mindestens ein Tensid, ausgewählt aus anionischen, nichtionischen, amphteren und kationischenTensiden, enthält..

8. Verfahren nach einem der vorhergehenden Ansprüche, **dadurch gekennzeichnet, dass** die wässrige Zusammensetzung, die mindestens ein Reduktionsmittel enthält, mindestens ein organisches Lösungsmittel als Penetrationsverstärker und/oder Harnstoff enthält.

9. Verfahren nach einem der vorhergehenden Ansprüche, **dadurch gekennzeichnet, dass** die wässrige Zusammensetzung, die mindestens ein Reduktionsmittel enthält, mindestens ein Haar konditionierendes Mittel enthält.

## Revendications

1. Procédé pour obtenir un produit pour la mise à forme permanente consistant en un récipient pressurisé et en une composition aqueuse comprenant au moins un réducteur **caractérisé en ce que**
a- le récipient (1) consistant en une paroi externe (2) ayant une fermeture (3) avec une valve (5) et un fond (4) avec un obturateur (6) qui contient un piston (7) non supporté déplaçable verticalement avec un élément de recouvrement (12) flexible en silicone et le support (11), le piston déplaçable divise le volume entier du récipient en deux volumes complètement séparés (8 et 9), l'un (8) étant rempli avec une composition aqueuse à permanente et l'autre (9), étant la partie inférieure, est rempli d'un gaz ininflammable exempt d'oxygène pour augmenter la pression dans le récipient est tout d'abord placé dans un espace de remplissage,
b-grâce à une buse de remplissage allongeable entrant dans le récipient par la zone de fermeture, le piston déplaçable (7) est poussé jusqu'au fond du récipient puis est suivi d'une purge du contenu du récipient avec un gaz exempt d'oxygène préférentiellement le même que le gaz de pressurisation et est choisi parmi l'azote et le dioxyde de carbone, à travers une buse qui peut être la même ou différente de la buse de remplissage,
c-à travers l'ouverture (6) au fond (4) du récipient la partie inférieure du récipient est purgée avec un gaz exempt d'oxygène préférentiellement le gaz de pressurisation et est choisi parmi de l'azote et du dioxyde de carbone,
d- pousser le piston (7) de nouveau vers le bas à travers l'ouverture avec une buse allongeable et remplir de composition aqueuse comprenant au moins un réducteur déjà produit dans la partie supérieure du récipient (8),
e-purger encore avec un gaz ininflammable exempt d'oxygène l'espace supérieure du récipient restant après remplissage de composition,
f-placer la fermeture (3) et la valve (5) sur le dessus du récipient, et
g- du gaz pressurisé est injecté dans la partie inférieure (9) et l'obturateur (bouchon) (6) est placé.

2. Procédé selon la revendication 1, **caractérisé en ce que** les étapes b et c sont réalisées dans l'ordre inverse.

3. Procédé selon l'une des revendications 1 et 2, **caractérisé en ce que** le gaz ininflammable exempt d'oxygène est choisi parmi l'azote, le dioxyde de carbone et le protoxyde d'azote (N₂O).

4. Procédé selon les revendications 1, 2 et 3, **caractérisé en ce que** le réducteur dans la composition aqueuse est choisi parmi l'acide thioglycolique, la cystéine, l'acide thiolactique, la cystamine.

5. Procédé selon les revendications 1 à 4, **caractérisé en ce que** la composition aqueuse comprenant au moins un réducteur a une viscosité inférieure à 5000 mPa.s mesurée à 20°C avec un viscosimètre de Brookfield.

6. Procédé selon l'une quelconque des revendications précédentes, **caractérisé en ce que** la composition aqueuse comprenant au moins un réducteur a un pH compris entre 5 et 10.

7. Procédé selon l'une quelconque des revendications précédentes, **caractérisé en ce que** la composition aqueuse comprenant au moins un réducteur comprend au moins un surfactant choisi parmi les formes anioniques, non ioniques, ampholytes et cationiques.

8. Procédé selon l'une quelconque des revendications précédentes, **caractérisé en ce que** la composition aqueuse comprenant au moins un réducteur comprend au moins un solvant organique en tant qu'améliorateur de pénétration et/ou de l'urée.

9. Procédé selon l'une quelconque des revendications précédentes, **caractérisé en ce que** la composition aqueuse comprenant au moins un réducteur comprend un agent de conditionnement des cheveux.
